Europäisches Patentamt

European Patent Office

Office européen des brevets

(11) Veröffentlichungsnummer : **0 332 578 B1**

(19)

(12) # EUROPÄISCHE PATENTSCHRIFT

(45) Veröffentlichungstag der Patentschrift :
**25.11.92 Patentblatt 92/48**

(21) Anmeldenummer : **89810154.8**

(22) Anmeldetag : **28.02.89**

(51) Int. Cl.$^5$ : **C07C 211/63,** C07C 211/62,
C07C 211/64, C07C 215/90,
C07F 9/54, C07D 265/30,
C07D 295/02, C07D 233/58,
C09D 5/14

(54) **Biocid wirksame Phosphonium-Verbindungen.**

(30) Priorität : **08.03.88 CH 862/88**

(43) Veröffentlichungstag der Anmeldung :
**13.09.89 Patentblatt 89/37**

(45) Bekanntmachung des Hinweises auf die
Patenterteilung :
**25.11.92 Patentblatt 92/48**

(84) Benannte Vertragsstaaten :
**DE ES FR GB IT NL SE**

(56) Entgegenhaltungen :
FR-A- 1 058 618
CHEMICAL ABSTRACTS, Band 107, 1987, Referat Nr. 177897b in Verbindung mit Formelregister Band 87, Seite 2776F, Spalte 3,Zeile 45,
Columbus, Ohio, USA; V. I. SHLYAKHOV et al,
"Imparting of antibacterial properties to polyester fabrics"

(56) Entgegenhaltungen :
CHEMICAL ABSTRACTS, Band 107, 1987, Referat Nr. 15534d in Verbindung mit Formelregister Band 87, Seite 2945F, Spalte 3,Zeilen 1-3,
Columbus, Ohio, USA; A. YAMAMOTO et al,
"Charge-endowing materials for electrostatic
image development"
CHEMICAL ABSTRACTS, Band 107, 1987, Referat Nr. 168042Y, Columbus, Ohio, USA; K.
TANAKA et al, "Charge-donating materials
forelectrostatic image development"

(73) Patentinhaber : **CIBA-GEIGY AG**
**Klybeckstrasse 141**
**CH-4002 Basel (CH)**

(72) Erfinder : **Wehner, Wolfgang, Dr.**
**Wetzbach 34**
**W-6144 Zwingenberg (DE)**
Erfinder : **Lorenz, Joachim, Dr.**
**Im Tiefen Weg 17**
**W-6140 Bensheim (DE)**
Erfinder : **Grade, Reinhardt, Dr.**
**Tulpenweg 11**
**W-6140 Bensheim (DE)**

EP 0 332 578 B1

## Beschreibung

Die Erfindung betrifft neue biozid wirksame Verbindungen der Gruppe der quaternären Ammonium- und Phosphoniumbasen.

Es ist aus der FR-PS 1 058 618 bekannt, quaternäre Ammoniumfluorborate, wie Dodecyldimethylbenzylammoniumfluoroborat als Fäulnis verhinderndes Mittel auf textile Fasern aufzubringen.

Aus der JP-PS 61-258,270 ist eine Verbindung der Art $N^{\oplus}Et_2$ Benzyl $C_{12}H_{25}$ $PF_6^{\ominus}$ als Ladungsträger für die elektrostatische Bildentwicklung bekannt.

Die GB-PS 994,881 beschreibt quaternäre Ammoniumfluoroborate, beispielsweise n-Alkyldimethylbenzylammoniumfluoroborate, als Katalysatoren für die Herstellung von wärmehärtenden Harzen.

In Chemical Abstracts, 1987, 107, Nr. 177897b werden Ammoniumverbindungen und deren biozide Wirksamkeit offenbart, während in Chemical Abstracts, 1987, 107, Nr. 15534d Phosphoniumverbindungen und deren Verwendung in der elektrostatischen Photographie beschrieben werden.

Es wurden nun überraschenderweise äusserst wirksame Biozide mit breitem biologischem Wirkungsspektrum aus der Reihe Phosphoniumbasen als Kation in Verbindung mit einem Anion aus der Gruppe der komplexen Fluoranionen gefunden.

Die vorliegende Erfindung betrifft Verbindungen der Formel

$$R^1_3 \ Y^{\oplus} \ R^2 \ X^{\ominus} \qquad (I),$$

worin die $R^1$ gleich oder verschieden sind und $C_3$-$C_6$-Alkyl, Phenyl, $C_1$-$C_2$ alkylsubstituiertes Phenyl oder $C_5$-$C_7$-Cycloalkyl, Benzyl, Tolyl oder Hydroxy-$C_1$-$C_4$-alkyl bedeuten
und
$R^2$ geradkettiges oder verzweigtes $C_8$-$C_{22}$-Alkyl ist
und
Y die Beteutung von P hat
und
X gleich $BF_4$ oder $PF_6$ ist.

Zweckmässig sind Verbindungen der Frormel I, in denen die $R^1$ die Bedeutung von $C_3$-$C_6$-Alkyl und besonders von i-Propyl, n-Propyl, n-Butyl oder Phenyl, Cyclohexyl, Benzyl oder Hydroxyethyl haben.

Die Erfindung betrifft vorzugsweise Verbindungen der Formel I, worin Y die Bedeutung von P hat und die $R^1$ gleich oder verschieden sind und i-Propyl, n-Butyl, Phenyl oder Cyclohexyl bedeuten
und
$R^2$ geradkettiges oder verzweigtes $C_8$-$C_{22}$-Alkyl ist
und
X gleich $BF_4$ oder $PF_6$ ist.

In den Verbindungen der Formel I ist $R^2$ ein geradkettiger oder verzweigter $C_8$-$C_{22}$-Alkylrest und kann beispielsweise n-Octyl, 2-Ethylhexyl, 1-Methylheptyl, 1,1,3-Trimethylhexyl, Decyl, Undecyl, 1-Methylundecyl, Dodecyl, Tridecyl, Tetradecyl, Pentadecyl, Hexadecyl, Heptadecyl, Octadecyl, Nonadecyl, Eicosyl, Henicosyl oder Docosyl darstellen. Zweckmässig sind die Alkylreste mit 10 bis 16 C-Atomen, so beispielsweise die Decyl-, Undecyl-, Dodecyl-, Tridecyl-, Tetradecyl-, Pentadecyl- und Hexadecylreste und bevorzugt sind Alkylreste mit 12 bis 14 C-Atomen. Als besonders bevorzugt können der n-Dodecylrest und der n-Tetradecylrest gelten.

Bevorzugte erfindungsgemässe Verbindungen der Formel I sind solche, in denen $R^1$ n-Butyl ist.

In erfindungsgemässen Verbindungen der Formel (I) bedeutet X vorzugsweise $BF_4$.

Beispiele für erfindungsgemässe Verbindungen der Formel (I) sind:

$(i$-$C_3H_7)_3 \ Y^{\oplus}R^2X^{\ominus}$

$(i$-$C_3H_7)_2 \ (C_6H_5) \ Y^{\oplus}R^2X^{\ominus}$

$(i$-$C_3H_7) \ (C_6H_5)_2 \ Y^{\oplus}R^2X^{\ominus}$

$(i$-$C_4H_9)_3 \ Y^{\oplus}R^2X^{\ominus}$

$(i$-$C_4H_9)_2 \ (C_6H_5) \ Y^{\oplus}R^2X^{\ominus}$

$(i$-$C_4H_9) \ (C_6H_5)_2 \ Y^{\oplus}R^2X^{\ominus}$

$(i$-$C_3H_7)_2 \ (C_6H_5) \ Y^{\oplus}R^2X^{\ominus}$

$(i$-$C_4H_9)_2 \ (C_6H_5) \ Y^{\oplus}R^2X^{\ominus}$

$(i$-$C_4H_9) \ (C_6H_5)_2 \ Y^{\oplus}R^2X^{\ominus}$

$(C_6H_5)_3 \ Y^{\oplus}R^2X^{\ominus}$

$(n$-$C_3H_7)_3 \ Y^{\oplus}R^2X^{\ominus}$

$(n$-$C_3H_7)_2(n$-$C_4H_9)Y^{\oplus}R^2X^{\ominus}$

$(n$-$C_3H_7) \ (n$-$C_4H_9)_2 \ Y^{\oplus}R^2X^{\ominus}$

$(n$-$C_4H_9)_3 \ Y^{\oplus}R^2X^{\ominus}$

wobei Y, $R^2$ und X die oben angegebene Bedeutung haben.

Beispiele für bevorzugte Verbindungen sind:

$(n-C_4H_9)_3\,P^{\oplus}(n-C_{14}H_{29})PF_6{}^{\ominus}$,

$(C_6H_5)_3\,P^{\oplus}(n-C_{12}H_{25})BF_4{}^{\ominus}$.

Besonders bevorzugt ist die Verbindung der Formel

$$(n-C_4H_9)_3P^{\oplus}(n-C_{14}H_{28})BF_4{}^{\ominus}.$$

Die erfindungsgemässen Verbindungen können auf an sich bekannte Weise hergestellt werden.

Es werden beispielsweise entsprechende Phosphoniumhalogenide mit Metall-, z.B. Alkalimetall-, oder mit Ammoniumfluoroboraten oder -phosphaten zu den entsprechenden neuen erfindungsgemässen Phosphoniumfluorsalzen umgesetzt.

Beispielsweise kann Ammoniumtetrafluoroborat bzw. Bleitetrafluoroborat oder Ammoniumhexafluorphosphat mit einem quaternären Phosphoniumhalogenid $R^1_3\,YR^2$ Hal (Hal = Halogen) umgesetzt werden. Die Reaktionsbedingungen sind dabei nicht kritisch und die Reaktion kann z.B. bei Normaldruck und Raumtemperatur und beispielsweise mit Wasser als Lösungsmittel ausgeführt werden.

Die Ausgangsmaterialien sind ebenfalls an sich bekannt und stellen zum Teil Handelsprodukte dar.

Die erfindungsgemässen Verbindungen sind ausgezeichnete Biozide und haben ein breites biologisches Wirkungsspektrum, so dass sie generell für den technischen Materialschutz eingesetzt werden können. Insbesondere wirken sie gegen Bakterien, Fungi und Algen, Protozoen, Mollusken, Muscheln, Balaniden, Bryocoen, Hydroiden usw.

Die Verbindungen der Formel I werden daher erfindungsgemäss als Biocide verwendet, insbesondere gegen die vorstehend genannten Organismen, z.B. als technische Biocide im Materialschutz.

Entsprechend sind die erfindungsgemässen Verbindungen geeignet - als Konservierungsmittel für technische Lösungen - als Zusätze zu Baustoffen, vorzugsweise zu Mörteln, Putzen (Innenputz, Aussenputz, Estrich usw.) oder zu hydraulische Bindemittel enthaltenden Mischungen, wie Beton, - als Zusätze zu Metallbearbeitungsflüssigkeiten, vorzugsweise zu Bohr- und Schneidölen, ferner auch zu Walzwerk- und Schmiede-Trenn- und Schmierstoffen, - als Zusatz zu Anstrichstoffen, zweckmässig zu Farben und Lacken, vorzugsweise zu Dispersionfarbstoffen, - als aktives Medium in Fäulnis hemmenden oder verhindernden Anstrichen, sogenannten Anti-Fouling-Anstrichen, zur bioziden Ausrüstung von Oberflächenbeschichtungen allgemein und von Holz, Kunststoffen, Polymermaterialien, Papier, Leder, Textilien im besonderen - zur oberflächlichen Behandlung von oder zur Einarbeitung in Baumaterialien und Bauelemente aus Polymermaterial, - als Antischleimmittel in Wassersystemen, vorzugsweise in Systemen für Kühl-Wasser und auch vorzugsweise im Brauch-Wasser, insbesondere der celluloseverarbeitenden Industrie, wie der Papierindustrie, und schliesslich - zur Desinfektion.

Eine besonders bevorzugte Verwendung der erfindungsgemässen Verbindungen ist in Anti-Fouling-Anstrichen und dabei besonders für Anstriche an in Meerwasser untergetauchten Objekten.

Die erfindungsgemässen Verbindungen gelangen insbesondere überall dort zur Anwendung, wo Objekte, welche gegen den Bewuchs durch Pilze und Algen und Befall durch Balaniden, Bryocoen, Hydroiden, Mollusken, Protozoen, Muscheln und Bakterien geschützt werden sollen, dem Meerwasser ausgesetzt sind. Es handelt sich dabei insbesondere um Schiffskörper, Wasserbauten, Bojen oder Fischernetze, jedoch auch Kühl- und Rohrsysteme, welche von Meerwasser umströmt oder durchflossen werden. Allgemein schützen die erfindungsgemässen Verbindungen alle Materialien, welche mit Meerwasser in Kontakt treten können, vor Bewuchs bzw. Befall durch die vorgenannten Organismen, z.B. Holz, Zellstoff, Textilien und Leder, Farben, Lacke, z.B. Antifoulingfarben und ähnliche Beschichtungsstoffe, optische und andere Gläser, Kunststoffe, Gummi und Klebestoffe, Metalle und mineralische Baustoffe, sowie andere Materialien.

Die Verbindungen werden je nach Verwendungszweck in den dem Fachmann bekannten Konzentrationsbereichen eingesetzt. Die Grenzen der gebräuchlichen Konzentrationen sind durch folgende Werte gegeben: Während in Kühlwasser bereits Konzentrationen im ppm-Bereich genügen, so sind in Antifoulingrezepturen Konzentrationen bis 40 Gew.-% üblich.

Die Verbindungen können in reiner Form oder zusammen mit Trägerstoffen als Stäube-, Streu- oder Nebelmittel appliziert werden. Sie können auch in flüssigen Medien gelöst oder suspendiert werden, wobei gegebenenfalls zur Bildung von gleichmässigen Dispersionen Netzmittel oder Emulgiermittel die gleichmässige Verteilung des Wirkstoffs fördern kann. Es können weitere Biocide hinzugefügt werden.

Ein besonders bevorzugter Anwendungsbereich sind Schutzanstrichstoffe, insbesondere Antifoulingfarben, die neben den üblichen Grund- und Zusatzstoffen 0,5-40 Gew.-%, vorzugsweise 3 bis 15 Gew.-%, bezogen auf die Gesamtmischung, wenigstens einer Verbindung der Formel I, enthalten.

Ueblich Grundstoffe für Antifoulingfarben sind die als Bindemittel bezeichneten und dem Fachmann bekannten Lackrohstoffe, wie natürliche und synthetische Harze, homo- und copolymere Produkte mit den Monomeren Vinylchlorid, Vinylidenchlorid, Styrol, Vinyltoluol, Vinylestern, Vinylalkoholen, Acrylsäure und Methacrylsäure sowie deren Estern, Polyester- und Polyamidharze, ferner Chlorkautschuk, natürlicher und synthe-

EP 0 332 578 B1

tischer Kautschuk, gegebenenfalls chloriert oder cyclisiert, dann auch Reaktionsharze wie Epoxidharze, Polyurethanharze, ungesättigte Polyester, die gegebenenfalls durch Zusatz von Härtern in filmbildende höhermolekulare Produkte übergeführt werden können.

Die Bindemittel können flüssig sein oder in gelöster Form vorliegen. Bei gelösten Bindemitteln, auch Thermoplasten, kann ein Schutzfilm auch durch Verdampfen des Lösungsmittels gebildet werden. Feste Beschichtungsmittel können z.B. im Pulverbeschichtungsverfahren auf Gegenstände aufgebracht werden. Weitere übliche Grundstoffe sind z.B. Teer, Modifikatoren, Farbstoffe, anorganische oder organische Pigmente, Füllstoffe und Härter.

Die erfindungsgemässen Verbindungen können schliesslich auch in elastomeren Beschichtungen sowie auch in Silikon-Elastomeren und Fluor enthaltenden Polymeren Anwendung finden.

In der Praxis werden Wirkstoffe vielfach in Kombination mit anderen Biociden eingesetzt. Auch die erfindungsgemässen Verbindungen können mit anderen Biociden kombiniert werden. Bei Antifoulingfarben erweisen sich Kombinationen von Produkten oft als vorteilhaft. So können die erfindungsgemässen Verbindungen z.B. in Kombination mit $Cu_2O$, CuSCN, Zinkoxid, Triorganozinnverbindungen, wie Tributylzinnfluorid oder Triphenylzinnchlorid, metallischem Kupfer oder Triazinen oder allgemein mit solchen Verbindungen, die dem Fachmann als gegen tierischen oder pflanzlichen Bewuchs wirksam bekannt sind, verwendet werden.

Eine weitere Verwendungsform der Verbindungen nach der Erfindung ist das Einarbeiten in Kunststoffe oder natürliche oder synthetische Kautschuke, oder das Aufbringen auf Oberflächen von Formkörpern aus diesen Kunststoffen, z.B. Polyvinylchloriden und deren Co- und Mischpolymerisaten, Polyalkylenen, Polyacrylaten, Polystyrolen, Copolymeren davon, Polyurethanen oder Polyisocyanaten, Polyestern, Epoxidharzen usw.

Sinnvoll ist die Verwendung insbesondere bei Kunststoffen oder Polymermaterialien, die als Baumaterialien Verwendung finden und beispielsweise der Witterung ausgesetzt sind oder im Bereich von feuchtenden oder nässenden Bereichen eingesetzt werden. Dabei können beispielhaft Dachmaterialien oder Verkleidungen aus Polyvinylchlorid, Butylkautschuk, chloriertem Polyethylen, Polyisobutylen, Chloropren und Chloroisopren, EPDM sowie PVC-Mischpolymerisaten mit Vinylacetat oder Ethylvinylacetat, Polyacrylnitrilstyrol, gegebenenfalls in Mischung mit faserigen Füllstoffen (gegebenenfalls auch im Verschnitt mit Bitumen), oder geschäumte Polyvinylchloride oder Polystyrole, als Isolationsmaterialien gegen Wärme und Kälte, genannt werden.

Die erfindungsgemässen Verbindungen sind sowohl für genannte, als auch für weitere Anwendungen geeignet. Sie sind nicht hygroskopisch und thermostabil und haben eine sehr geringe Wasserlöslichkeit.

Die Erfindung umfasst auch Zusammensetzungen enthaltend wenigstens eine Verbindung der Formel I nach vorliegender Erfindung. Die Form und Art der jeweiligen Zusammensetzung, enthaltend die erfindungsgemässe Verbindung, richtet sich nach dem Einsatzzweck. Zweckmässig und bevorzugte Verbindungen, wie oben erwähnt, führen zu zweckmässigen und bevorzugten Zusammensetzungen.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen:

Stäubemittel und Streumittel (bis zu 10 %), Granulate, Umhüllungsgranulate, Imprägnierungsgranulate und Homogengranulate, Pellets (Körner) (1 bis 80 %).

Flüssige Aufarbeitungsformen:

a) in Wasser dispergierbare Wirkstoffkonzentrate:

Spritzpulver (wettable powders) und Pasten (25-90 % in der Handelspackung, 0,01 bis 15 % in gebrauchsfertiger Lösung), Emulsions- und Lösungskonzentrate (10 bis 50 %; 0,01 bis 15 % in gebrauchsfertiger Lösung);

b) Organische Lösungen (0,1 bis 20 %); Aerosole.

Die Erfindung umfasst somit ferner Mittel, welche die erfindungsgemässen Verbindungen enthalten, sowie die Verwendung der erfindungsgemässen Verbindungen und Mittel zur Bekämpfung von Schadorganismen, z.B. Bakterien, Fungi, Algen, Protozoen, Mollusken, Muscheln, Balaniden, Bryocoen, Hydroiden usw., insbesondere im Materialschutz.

Die erfindungsgemässen biociden Mittel können auch weitere Aktivsubstanzen enthalten.

Beispiele dafür sind:

a) Organo-Schwefelverbindungen, z.B. Methylen-dithiocyanat (MBT), Isothiazolone oder 3,5-Dimethyl-tetrahydro-1,3,5-2H-thiodiazin-2-thion (DMTT). Solche Substanzen werden insbesondere gegen Schleimbildung bei der Papierherstellung eingesetzt.

b) Chlorierte Phenole, wie Natrium-pentachlorphenolat. Solche Verbindungen zeichnen sich durch ein sehr breites Wirkungsspektrum aus.

c) Kupfersalze, wie Kupfersulfat und Kupfernitrat als zusätzliche Algicide.

d) 2,2-Dibrom-3-nitrilopropionamid (DBNPA) als Algicid, Fungicid und Bactericid.

e) Chlor und Brom als Algicide und Bactericide, welche besonders bei der Wasserbehandlung zum Einsatz

4

gelangen.

f) Chlordioxid, Chlorisocyanurate und Hypochlorite als Biocide, z.B. bei der Wasserbehandlung.

g) Triazine, z.B. 2-Methylthio-4-t-butylamino-6-cyclopropylamino-s-triazin, insbesondere als Algicide.

h) Triorganozinnverbindungen, z.B. Bis-tributylzinnoxid (TBTO), insbesondere als Molluscicide, Fungicide und Algicide.

i) Holzbiocide

    ia) Salzgemische auf Basis von

    Silicofluoriden, Hydrogenfluoriden, anorg. Borverbindungen, Chromaten, Fluoriden, Arsen (Oxid, Arsenate), Kupfersalze (Sulfat, Naphthenat), Zinn- und Zinksalzen, Quecksilberverbindungen.

    ib) Teerölpräparate

    ic) Organische Wirkstoffe, wie

    Pentachlorphenol, Phenol, DDT, Dieldrin, Lindan, Gammexan, chlorierte Naphthaline, Dichlorfluanid, Tributylzinnverbindungen, Pyrethroide, 3-Jod-2-propenyl-N-butylcarbamat, Furmecyclox.

j) Desinfektionsmittel

    ja) Phenol oder Phenolderivate

    jb) Formaldehyd und/oder sonstige Aldehyde bzw. Derivate

    jc) Chlor, organ. oder anorganische Substanzen mit aktivem Chlor

    jd) Amphotenside

    je) Quaternäre Oniumverbindungen.

Selbstverständlich können in solchen Formulierungen ausserdem noch weitere Substanzen und Hilfsmittel enthalten sein, wie sie üblicherweise in solchen Zubereitungen mitverwendet werden. Hierzu gehören z.B. kationische oder nichtionische oberflächenaktive Substanzen, Elektrolyte, Komplexbildner, Lösungsvermittler sowie Farb- und Duftstoffe. Diese Zusätze dienen beispielsweise zur Verbesserung des Netzvermögens, der Härtungsstabilität, zur Viskositätseinstellung und zur Erhöhung der Kältestabilität der Lösungen.

Bei der Verwendung in Dispersionsfarben und -putzen können die erfindungsgemässen Verbindungen mit einem weiteren Fungizid kombiniert werden. In der Wasserbehandlung sind Kombinationen mit einem Baktericid möglich, um schleimbildende Bakterien zu bekämpfen. Solche Kombinationen können anwendungstechnische Vorteile bringen. In vielen Fällen ist auch die Kombination mit weiteren Algiziden von Vorteil.

Die Erfindung umfasst auch Zusammensetzungen enthaltend a) einen Anstrichstoff und b) eine wirksame Menge wenigstens einer Verbindung der Formel (I) nach vorliegender Erfindung.

Die nachfolgenden Beispiele erläutern die Erfindung näher, ohne ihren Umfang zu begrenzen. Hierin angegebene Prozente (%) bedeuten Gewichtsprozente und Teile Gewichtsteile.

Beispiele:

### 1. Tri-n-butyl-n-tetradecyl-phosphonium-tetrafluoroborat

Zu 314,5 g (3,0 Mol) Ammoniumtetrafluoroborat in 5 l $H_2O$ tropft man unter kräftigem Rühren bei 2°C 2176 g einer 50%igen Lösung des Tri-n-butyl-n-tetradecylphosphoniumchlorides in Wasser zu, wobei ein dicker Niederschlag entsteht. Nach kurzem Absitzen wird abgesaugt, 3 x portionsweise mit 500 ml Wasser nachgewaschen und über $P_2O_5$ auf Gewichtskonstanz getrocknet.

Ausbeute: 1192 g, entsprechend 98 % der Theorie - Fp.: 39°C

### 2. Tri-n-butyl-n-tetradecyl-phosphonium-hexafluorophosphat

Herstellung erfolgt analog Beispiel 1, jedoch unter Verwendung einer äquivalenten Menge einer 50%igen wässrigen Ammoniumhexafluorophosphatlösung.

Ausbeute: 80 % - Fp.: 41°C

### 3. Triphenyl-n-dodecyl-phosphonium-tetrafluorborat

22,3 g (0,0143 Mol) einer 30%igen wässrigen Lösung von Triphenyl-n-dodecyl-phosphoniumchlorid werden mit 5,5 g (0,00715 Mol) einer 50%igen wässrigen Lösung von Bleitetrafluoroborat unter Rühren versetzt. Der entstandene Niederschlag wird mit 250 ml Methanol digeriert, vom Bleichlorid abfiltriert und die methanolische Lösung auf Rückstand eingeengt.

Ausbeute: quantitativ- gelbes hochviskoses Oel, $n_D^{20}$ = 1,5414.

Beispiel 4: Bestimmung der minimalen Hemmkonzentration gegen Bakterien

Die in Caso-Pepton-Bouillon (Merck) gewachsenen üNK's (über Nacht bebrütete Kulturen) der verschiedenen Bakterienstämme:

A) Proteus vulgaris, B) Pseudomonas aeruginosa, C) Enterobacter aerogenes, D) Serratia marcescens, E) Alcaligenes denitrificans, F) Bacillus subtilis,

werden jeweils in Saline 1/1000 verdünnt. Von den Suspensionen wird soviel in Caso-Pepton-Bouillon gegeben, dass die Bakterien erneut auf 1/1000 verdünnt werden. Danach werden jeweils die in Tabelle 1 angeführten Verbindungen in Mengen von 30, 100 und 300 mg/l zugegeben. Nach einer Bebrütung von 24 Stunden bei 30°C im Schüttelwasserbad wird nach Trübung ausgewertet. Die minimale Hemmkonzentration (MIC) ist die Konzentration, bei der die Bouillon nicht durch Bakterienwuchs trüb wird.

Die Ergebnisse sind in der folgenden Tabelle veranschaulicht:

Tabelle 1: Bestimmung der MIC gegen Bakterien (Konz. mg/l)

| Verbindung aus Beispiel | S t a m m | | | | | |
|---|---|---|---|---|---|---|
| | A | B | C | D | E | F |
| 1 | 30 | 100 | 30 | 30 | 30 | 30 |
| 3 | 30 | 30 | 30 | 30 | 30 | 30 |

Aus der Tabelle ist die gute wuchshemmende Wirkung der Verbindungen auch gegen die schwierig zu bekämpfenden gramnegativen Bakterien zu erkennen.

Beispiel 5: Bestimmung der minimalen Hemmkonzentration (MIC) gegen Pilze

Stämme:
A) Aspergillus niger
B) Sacharomyces cerevisiae
C) Penicillium funiculosum
D) Chaetomium globosum
E) Aureobasidium pullulans
F) Coniophora puteana

Die Untersuchung wird mit dem bekannten Agar-Inkorporations-Test im Malzextrakt-Agar (Merck) durchgeführt. Zur Hemmung werden jeweils soviel an den verschiedenen Verbindungen zugegeben, dass Konzentrationen von 10, 50 und 100 mg/l im Agar resultieren. Die zur Hemmung des Wuchses der Pilze (von aufgetropften Pilzsporen ausgehend) benötigten Konzentrationen (mg/l) sind in der Tabelle 2 veranschaulicht.

Tabelle 2: Bestimmung der MIC gegen Pilze (Konz. mg/l)

| Verbindung aus Beispiel | S t a m m | | | | | |
|---|---|---|---|---|---|---|
| | A | B | C | D | E | F |
| 1 | 50 | 10 | 10 | 10 | 50 | 10 |
| 3 | 100 | 50 | 10 | 50 | 10 | 50 |
| 2 | 100 | 10 | 10 | 100 | 50 | 100 |

getestete Konzentration 10, 50, 100 mg/l

Aus der Tabelle 2 ist zu erkennen, dass die Verbindungen auch hervorragende Fungicide sind.

Beispiel 6: Verwendung der Verbindungen als Antifouling-Biocide

Zur Prüfung der Wirksamkeit gegenüber Bewuchs an im Meer untergetauchten Objekten wird die Verbindung gemäss Beispiel 1 in Antifouling-Farben eingearbeitet. Hergestellt wird eine Antifouling-Farbe, wie sie dem Fachmann bekannt ist, auf Basis Vinylchloridmischpolymerisat/Kolophonium (2/1 Gewichtsteile) mit ca. 40 % Pigmentvolumenkonzentration und eine zweite Antifouling-Farbe ähnlichen Aufbaues, jedoch mit höherem Kolophonium-Anteil (Vinylchloridmischpolymerisat:Kolophonium = 1:1). In diese Farben werden die Verbindung gemäss Beispiel 1 eingearbeitet, auf Probeplatten aufgestrichen und nach dem Trocknen (10 Tage) in der Nordsee ausgehängt. Als Vergleich werden die entsprechenden Farben, jedoch ohne Zusatz des Produktes, ebenso ausgelegt. In einer Bewertungsskala von 0 (= völlig bewachsen) bis 10 (= völlig bewuchsfrei) ergeben sich nach 6 1/2 wöchiger Auslagerung folgende Ergebnisse (Bewuchs durch Hydroids = HY; Bewuchs durch Barnacles = BA):

Vinyl/Kolophonium 2:1: ohne Zusatz HY 5, BA 5
Vinyl/Kolophonium 2:1: mit Zusatz HY 8, BA 8
Vinyl/Kolophonium 1:1: ohne Zusatz HY 0, BA 0
Vinyl/Kolophonium 1:1: mit Zusatz HY 4, BA 10

Beispiel 7: Verwendung als Algizid in Polymermaterial

Zur Prüfung der Wirksamkeit gegen Algenbefall werden in aus Polyisobutylen bestehende Dachbahnen verschiedene, aus Tabelle 3 ersichtliche Konzentrationen der Verbindung gemäss Beispiel 1 eingearbeitet. Als Vergleich wird eine Dachbahn ohne Biocid untersucht.

Von jedem Teststreifen wird ein 1 cm$^2$ grosses Stück Folie 5 Tage gewässert, je auf eine Algen-Agarplatte gelegt und mit 0,1 ml einer 1+1+1-Mischung aus 14tägigen Kulturen der Algen

Chlorella vulgaris und
Chlorella spec. (aus Schadenfällen in der Praxis isoliert) und
Scenedesmus obliques,
die 1/10 in Saline verdünnt worden waren, angeimpft.

Nach 3 Wochen werden die Platten ausgewertet, die Folien auf frischen Agar gelegt und erneut geimpft.

Die Auswertung des Bewuchses nach 3 und 6 Wochen unter künstlicher Belichtung wird nach folgendem Schema durchgeführt.

1 = Bewuchs der Folie
2 = teilweise Wuchs auf der Folie
3 = Wuchs bis an den Folienrand
4 = Hemmhof um die Folie < 5 mm
5 = Hemmhof um die Folie > 5 mm

Folgendes Ergebnis wird erhalten:

Tabelle 3

|  |  | Algenbewuchs nach | |
|  |  | 3 | 6 Wochen |
|---|---|---|---|
| Folie mit Zusatz der | 0,1 | 4 | 4 |
| Verbindung gemäss | 0,5 | 4 | 4 |
| Beispiel 1 in Gew.-% | 1 | 4 | 4 |
| Vergleichsfolie ohne Zusatz |  | 2-3 | 2 |

Beispiel 8

Beispiel 7 wird wiederholt, jedoch an Stelle der angegebenen Konzentrationen an Verbindung gemäss Beispiel 1 allein wird eine Kombination aus 1 Gew. % der Verbindung gemäss Beispiel 1 und 0,07 Gew. % 2-Methylthio-4-t-butylamino-6-cyclopropylamino-s-triazin eingesetzt. Die erhaltenen Resultate sind der Tabelle 4 zu entnehmen.

Tabelle 4

|  | Algenbewuchs nach | |
|  | 3 | 6 Wochen |
| --- | --- | --- |
| Folie mit Zusatz von 1 % der Verbindung gemäss Beispiel 1 + 0,07 % 2-Methylthio-4-t-butylamino-6-cyclo-propylamino-s-triazin | 5 | 5 |
| Vergleichsfolie ohne Zusatz | 2-3 | 2 |

Beispiel 9: Verwendung als Fungizid im Holz

Bestimmung der Grenze der Wirksamkeit gegenüber holzzerstörenden Basidiomyceten, die auf Agar gezüchtet werden (in Anlehnung an EN 113). Das in dieser Europäischen Norm beschriebene Prüfverfahren ist ein Laboratoriumsverfahren, das eine Grundlage für die Beurteilung der Wirksamkeit eines Holzschutzmittels gegenüber holzzerstörenden Basidiomyceten bildet. Es ermöglicht die Grenzkonzentration zu bestimmen, von der ab eine anfällige Holzart nach der Tränkung unter den Versuchsbedingungen als ausreichend geschützt angesehen werden kann.

Prüfpilze Die Prüfpilze sind nachstehend angegeben:

- Coniophora puteana (Schumacher ex Fries) Karsten, Stamm FPRL 11E, für Nadelhölzer = Stamm 91
- Poria placenta (Fries) Cooke sensu J. Eriksson, Stamm SPRL 280, für Nadelhölzer = Stamm 96
- Gloeophyllum trabeum (Persoon ex Fries) Murrill, Stamm BAM Ebw. 109, für Nadelhölzer = Stamm 94
Die Zucht der Stämme und die Zusammensetzung des Nährmedium erfolgte wie in EN 113 beschrieben.
Es werden Lösungen von Isopropanol/Wasser (1:1), enthaltend 0,2 %, 0,5 %, 1 %, 2 %, und 5 % Aktivsubstanz nach Beispiel 1, angesetzt.
Die Probehölzer aus Kiefer, Nennmasse: 50 mm x 25 mm x 15 mm werden, wie in EN 113 beschrieben, gewonnen und vorbereitet.
Die Tränkung nach EN 113 sichert die vollständige Durchtränkung der Probehölzer mit der Holzschutzmittellösung.
Die aufgenommene Schutzmittelmenge wird in Gewicht je Volumeneinheit des Holzes umgerechnet.
Nach dem Tränken werden die Probehölzer 4 Wochen lang getrocknet, konditioniert und zur Sterilisierung in Folien mit Elektronen (Strahlenenergie 2,5 MeV, Mindestdosis 25 k Gy) sterilisiert.
Der eigentliche Pilzversuch wird, wie in EN 113 beschrieben, durchgeführt (Versuchsdauer 16 Wochen).
Bei Versuchsende werden die Probehölzer aus den Versuchsgefässen herausgenommmen und von anhaftendem Pilzmycel befreit.
Die Grenze der Wirksamkeit eines Holzschutzmittels wird durch die beiden Holzschutzmittelmengen festgelegt, die
- der niedrigsten Konzentration, die das Holz schützt
- der nächstniedrigen Konzentration der Reihe, bei der das Holz nicht mehr ausreichend geschützt ist, entsprechen.
Der durch das Holzschutzmittel erreichte Schutz wird bei einer gegebenen Konzentration als ausreichend angesehen, wenn
- der mittlere korrigierte Masseverlust der Probehölzer unter 3 % liegt
- höchstens ein Probeholz einen Masseverlust von mehr als 3 % aber weniger als 5 % aufweist.
Die Grenze der Wirksamkeit wird durch die begrenzenden Werte in kg Schutzmittel je m$^3$ Holz für jede Pilzart angegeben. Die entsprechenden Konzentrationen des Schutzmittels in dem jeweiligen Lösungs- oder Verdünnungsmittel sind angegeben.

8

| Stamm | Nr. | Wirksame Konzentration (%) | Aufnahme kg/m$^3$ |
|---|---|---|---|
| Coniophora puteana FRRL 11 E | 91 | 0,5-1,0 | >3,0-<6,0 |
| Poria placenta FRRL 280 | 96 | 1,0-2,0 | >5,0-<10,0 |
| Gleophyllum trabeum BAM, EbW 109 | 94 | ≤0,2 | ≤1,0 |

Wie aus der Tabelle zu erkennen, ist die Aktivsubstanz nach Beispiel 1 hoch wirksam gegen die hier untersuchten holzzerstörenden Pilze.

**Patentansprüche**

1. Verbindungen der Formel

$$R^1_3 \, Y^\oplus \, R^2 \, X^\ominus \qquad (I),$$

worin die $R^1$ gleich oder verschieden sind und $C_3$-$C_6$-Alkyl, Phenyl, $C_1$-$C_2$ alkylsubstituiertes Phenyl oder $C_5$-$C_7$-Cycloalkyl, Benzyl, Tolyl oder Hydroxy-$C_1$-$C_4$-alkyl bedeuten
und
$R^2$ geradkettiges oder verzweigtes $C_8$-$C_{22}$-Alkyl ist
und
Y die Bedeutung von P hat
und
X gleich $BF_4$ oder $PF_6$ ist.

2. Verbindungen nach Anspruch 1 der Formel I, in denen die $R^1$ i-Propyl, n-Propyl, n-Butyl, Phenyl, Cyclohexyl, Benzyl oder Hydroxyethyl bedeuten.

3. Verbindungen nach Anspruch 1 der Formel I, wobei
Y die Bedeutung von P hat und die $R^1$ gleich oder verschieden sind und i-Propyl, n-Butyl, Phenyl oder Cyclohexyl bedeuten
und
$R^2$ geradkettigtes oder verzweigtes $C_8$-$C_{22}$-Alkyl ist
und
X gleich $BF_4$ oder $PF_6$ ist.

4. Verbindungen nach Anspruch 1 der Formel I, wobei $R^1$ n-Butyl ist.

5. Verbindungen nach einem der Ansprüche 1 bis 4 der Formel I, wobei X = $BF_4$ ist.

6. Verbindungen nach einem der Ansprüche 1 bis 5 der Formel I, wobei $R^2$ $C_{10}$-$C_{16}$-Alkyl und vorzugsweise $C_{12}$-$C_{14}$-Alkyl ist.

7. Verbindungen nach Anspruch 1 der Formeln

$$(n\text{-}C_4H_9)_3 \, P^\oplus (n\text{-}C_{14}H_{29}) \, BF_4^{\ominus},$$
$$(n\text{-}C_4H_9)_3 \, P^\oplus (n\text{-}C_{14}H_{29}) \, PF_6^{\ominus} \text{ und}$$
$$(C_6H_5)_3 \, P^\oplus (n\text{-}C_{12}H_{25}) \, BF_4^{\ominus}.$$

8. Zusammensetzungen enthaltend
   a) einen Anstrichstoff und
   b) wenigstens eine Verbindung der Formel (I) nach Anspruch 1.

9. Zusammensetzungen enthaltend
   a) ein Polymermaterial in Form von Baumaterial und
   b) wenigstens eine Verbindung der Formel (I) nach Anspruch 1.

**10.** Verwendung der Verbindungen der Formel I nach Anspruch 1 als Biocide.

**11.** Verwendung nach Anspruch 10 als Wirkstoff gegen Bakterien, Algen und Fungi, Muscheln, Protozoen, Balaniden, Bryocoen, Hydroiden und Mollusken.

**12.** Verwendung der Verbindungen der Formel (I) nach Anspruch 1 als Konservierungsmittel für technische Lösungen, als Zusätze zu Baustoffen, vorzugsweise zu Mörtel, Putzen oder Beton, als Zusätze zu Metallbearbeitungsflüssigkeiten, vorzugsweise zu Bohr- und Schneideölen, als Zusatz zu Anstrichstoffen, zweckmässig zu Farben und Lacken, vorzugsweise zu Dispersionsfarben, in Anti-Fouling-Anstrichen, zur bioziden Ausrüstung von Oberflächenbeschichtungen, von Holz, Kunststoffen, Polymermaterialien, Papier, Leder, Textilien, zur oberflächlichen Behandlung von oder zur Einarbeitung in Baumaterialien und Bauelemente aus Polymermaterial, als Antischleimmittel in Wassersystemen, vorzugsweise im Kühlwasser oder vorzugsweise im Brauchwasser der celluloseverarbeitenden Industrie und zur Desinfektion.

**13.** Verwendung nach Anspruch 12 in Anti-Fouling-Anstrichen an im Meerwasser untergetauchten Objekten.

**14.** Verwendung nach Anspruch 10 von Verbindungen der Formel I in Kombination mit biozid wirksamen s-Triazinen, insbesondere mit 2-Methylthio-4-t-butylamino-6-cyclopropylamino-s-triazin.

**15.** Verfahren zum Schützen von technischen Materialien gegen den Befall durch Schadorganismen, dadurch gekennzeichnet, dass man diesen Materialien mindestens eine Verbindung der Formel I einverleibt oder auf diese Materialien aufbringt.


**Claims**

**1.** A compound of the formula

$$R^1_3Y^{\oplus}R^2X^{\ominus} \qquad (I)$$

in which the radicals $R^1$ are identical or different and are $C_3$-$C_6$alkyl, phenyl, $C_1$-$C_2$alkyl-substituted phenyl or $C_5$-$C_7$cycloalkyl, benzyl, tolyl or hydroxy-$C_1$-$C_4$alkyl and $R_2$ is straight-chain or branched $C_8$-$C_{22}$alkyl, and Y is P, and X is $BF_4$ or $PF_6$.

**2.** A compound according to claim 1 of the formula I, in which the radicals $R^1$ are i-propyl, n-propyl, n-butyl, phenyl, cyclohexyl, benzyl or hydroxyethyl.

**3.** A compound according to claim 1 of the formula I, in which Y is P and the radicals $R^1$ are identical or different and are i-propyl, n-butyl, phenyl or cyclohexyl, and $R^2$ is straight-chain or branched $C_8$-$C_{22}$alkyl, and X is $BF_4$ or $PF_6$.

**4.** A compound according to claim 1 of the formula I, in which $R^1$ is n-butyl.

**5.** A compound according to any one of claims 1 to 4 of the formula I, in which X is $BF_4$.

**6.** A compound according to any one of claims 1 to 5 of the formula I, in which $R^2$ is $C_{10}$-$C_{16}$alkyl, and preferably $C_{12}$-$C_{14}$alkyl.

**7.** A compound according to claim 1 of the formula

$$(n\text{-}C_4H_9)_3 \, P^{\oplus}(n\text{-}C_{14}H_{29}) \, BF_4^{\ominus},$$
$$(n\text{-}C_4H_9)_3 \, P^{\oplus}(n\text{-}C_{14}H_{29}) \, PF_6^{\ominus} \text{ or}$$
$$(C_6H_5)_3 \, P^{\oplus}(n\text{-}C_{12}H_{25}) \, BF_4^{\ominus}$$

**8.** A composition containing
   a) a paint and
   b) at least one compound of the formula (I) according to claim 1.

**9.** A composition containing
   a) a polymeric material in the form of building material and
   b) at least one compound of the formula (I) according to claim 1.

**10.** The use of a compound of the formula I according to claim 1 as a biocide.

11. The use according to claim 10 as an active compound against bacteria, algae and fungi, mussels, protozoa, balanids, bryozoa, hydroids and molluscs.

12. The use of a compound of the formula (I) according to claim 1 as a preservative for industrial solutions, as an additive to building materials, preferably to mortar, plaster or concrete, as an additive to metalworking liquids, preferably to drilling and cutting oils, as an additive to coating materials, advantageously to paints and varnishes, preferably to emulsion paints, in anti-fouling paints, for biocidal finishing of surface coatings, wood, plastics, polymeric materials, paper, leather and textiles, for surface treatment of or incorporation into building materials and building components of polymeric materials, as an anti-slime agent in water systems, preferably in cooling water or preferably in industrial water in the cellulose-processing industry and for disinfection.

13. The use according to claim 12 in anti-fouling paints in objects immersed in seawater.

14. The use according to claim 10 of a compound of the formula I in combination with a biocidally active s-triazine, in particular with 2-methylthio-4-t-butylamino-6-cyclopropylamino-s-triazine.

15. A method of preserving industrial materials against attack by harmful organisms, which comprises incorporating into these materials or applying to these materials at least one compound of the formula I.

**Revendications**

1. Composés répondant à la formule I :

$$R^1{}_3 \, Y^{\oplus} \, R^2 \, X^{\ominus} \qquad (I)$$

dans laquelle

les $R^1$ représentent chacun, indépendamment l'un de l'autre, un alkyle en $C_3$-$C_6$, un phényle, un phényle porteur d'un alkyle en $C_1$ ou $C_2$, un cycloalkyle en $C_5$-$C_7$, un benzyle, un tolyle ou un hydroxy-alkyle en $C_1$-$C_4$,

$R^2$ représente un alkyle en $C_8$-$C_{22}$ linéaire ou ramifié,

Y représente P et

X représente $BF_4$ ou $PF_6$.

2. Composés de formule I selon la revendication 1 dans lesquels les $R^1$ représentent chacun un radical isopropyle, n-propyle, n-butyle, phényle, cyclohexyle, benzyle ou hydroxy-éthyle.

3. Composés de formule I selon la revendication 1 dans lesquels Y représente P, les $R^1$ représentent chacun, indépendamment l'un de l'autre, un radical isopropyle, n-butyle, phényle ou cyclohexyle, $R^2$ représente un radical alkyle en $C_8$-$C_{22}$, linéaire ou ramifié, et X représente $BF_4$ ou $PF_6$.

4. Composés de formule I selon la revendication 1 dans lesquels $R^1$ représente un radical n-butyle.

5. Composés de formule I selon l'une quelconque des revendications 1 à 4, dans lesquels X représente $BF_4$.

6. Composés de formule I selon l'une quelconque des revendications 1 à 5, dans lesquels $R^2$ représente un alkyle en $C_{10}$-$C_{16}$, de préférence en $C_{12}$-$C_{14}$.

7. Composés de formule I selon la revendication 1 qui répondent aux formules suivantes :

$$(n\text{-}C_4H_9)_3 \, P^{\oplus}(n\text{-}C_{14}H_{29}) \, BF_4{}^{\ominus},$$
$$(n\text{-}C_4H_9)_3 \, P^{\oplus}(_n\text{-}C_{14}H_{29}) \, PF_6{}^{\ominus} \text{ et}$$
$$(C_6H_5)_3 \, P^{\oplus}(n\text{-}C_{12}H_{25}) \, BF_4{}^{\ominus}.$$

8. Compositions qui contiennent :
   a) un produit de revêtement et
   b) au moins un composé de formule I selon la revendication 1.

9. Compositions qui contiennent :
   a) une matière polymère sous la forme d'un matériau de construction et
   b) au moins un composé de formule I selon la revendication 1.

**10.** Application des composés de formule I selon la revendication 1 comme biocides.

**11.** Application selon la revendication 10 comme matières actives contre des bactéries, des algues, des mycètes, des coquillages, des protozoaires, des balanidés, des bryozoaires, des hydroïdes et des mollusques.

**12.** Application des composés de formule I selon la revendication 1 comme agents de conservation pour des solutions techniques, comme additifs à des matériaux de construction, de préférence des mortiers, des crépis ou du béton, comme additifs à des liquides pour l'usinage de métaux, en particulier à des huiles de forage et de coupe, comme additifs à des produits de revêtement, avantageusement à des peintures, des vernis ou des encres, de préférence à des peintures de dispersions et des peintures anti-salissures, pour la protection par des biocides de revêtements de surfaces, du bois, de matières plastiques, de matières polymères, du papier, du cuir ou de textiles, pour le traitement de surface de matériaux et éléments de contruction en polymères ou pour l'incorporation dans de tels matériaux et éléments, comme anti-mucilages dans des systèmes aqueux, en particulier dans des eaux de refroidissement ou, mieux, dans l'eau utilisée dans l'industrie travaillant la cellulose, et pour la désinfection.

**13.** Application selon la revendication 12 dans des peintures anti-salissures sur des objets immergés dans de l'eau de mer.

**14.** Application selon la revendication 10 de composés de formule I en association avec des s-triazines biocides, plus particulièrement avec la 2-méthylthio-4-tert-butyalmino-9-cyclopropylamino-1,3,5-triazine.

**15.** Procédé pour protéger des matériaux industriels contre l'attaque par des organismes nuisibles, procédé caractérisé en ce qu'on incorpore à ces matières ou on applique sur ces matières au moins un composé de formule I.